# EUROPEAN PATENT APPLICATION

(11) **EP 1 106 699 A1**
(43) Date of publication of application: **13.06.2001**
(21) Application number: 99203857.0
(22) Date of filing: 18.11.1999
(51) Int. Cl.: C12P 17/02

(54) **Biocatalytic epoxidation of vinylaromatic compounds**

(71) Applicant: Eidgenössische Technische Hochschule Zürich, 8093 Zürich (CH)
(72) Inventor: Schmid, Andreas, 8049 Zürich (CH); Witholt, Bernard, 8032 Zürich (CH); Feiten, Hans-Jürgen, 8004 Zürich (CH); Panke, Sven, 6133 VC Sittard (NL)
(74) Representative: Ottevangers, Sietse Ulbe

(57) **Abstract**

Methods for preparation of biocatalysts and processes for application of these biocatalysts for the preparation of selected optically active epoxides with high enantiomeric purity. The methods are based on the stereoselective insertion of an oxygen atom derived from molecular oxygen catalyzed by a microbial monooxygenase enzyme like styrene monooxygenase into vinylaromatic compounds like indene or styrene derivatives as an epoxide function. In particular, these methods are useful for the preparation of (1S,2R) indene oxide. The enzyme preparations can be used in isolated, purified form or as whole cell preparations in specific recombinant hosts expressing the respective genes of microbial monooxygenases like styrene monooxygenase. Methods for preparing isolated enzymes and recombinant hosts as well as application methods for these systems in organic synthesis are described. Biocatalytic reactions are performed in aqueous medium or in multiphase media possibly containing one or more of the following: a solid phase, an aqueous phase, an organic phase, or a gaseous phase.

## Description

### Field of the invention

The present invention is in the field of synthesis of chemical compounds, in particular the enantiospecific (or stereoselective) epoxidation of vinylaromatic compounds, such as indene (1), styrene and derivatives thereof, to produce specific enantiomers of aromatic oxiran compounds (further referred to herein as phenyl oxirans).

In particular, the invention relates to a biocatalytic method for the preparation of (1S,2R) indene oxide (2) and derivatives thereof, wherein an epoxide functional group is inserted stereoselectively into indene or respective phenylethylene compound. The products of these reactions may be valuable building blocks for pharmaceuticals. For example, (1S,2R) indene oxide is a key intermediate in the synthesis of indinavir (Crixivan®), a potent HIV-1 protease inhibitor used in the treatment of AIDS.

### Background of the Invention

Epoxides are highly interesting synthons in organic chemistry due to their high reactivity. This is especially true for single enantiomers as a basis to introduce chirality in an early stage in the multistep synthesis of a complex final product.

One example of such a synthon is (1S,2R) indene oxide. (1S,2R) indene oxide is used as a synthon for the preparation of *cis*-aminoindanol in a Ritter reaction (Senanayake et al., 1995, Tetrahedron Letters 36: 3993-3996). Alternatively, *cis*-aminoindanol can be prepared in a Ritter reaction from *cis* (1S,2R) indanediol (Reider, 1997, Chimia 51: 306-308). *Cis*-aminoindanol is a key precursor for the preparation of CRIXIVAN® (Reider, 1997, Chimia 51: 306-308). Crixivan is the world's most widely used protease inhibitor for the treatment of AIDS (Merck & Co Inc., 1988 Annual report p. 20).

(1S,2R) indene oxide (87% ee (enantiomeric excess, which represents the amount of one enantiomer relative to the total amount of both enantiomers formed)) is accessible by chemical epoxidation of indene with a Mn^{II}Salen complex as Jacobsen catalyst (Hughes D. et al, 1997, J. Org. Chem. 62: 2222). On the other hand, turnover numbers are significantly lower than for corresponding oxygenases (Jacobsen E.N., 1993, Catalytic Asymmetric Synthesis, VCH New York, ed. Iwao Ojima, 159-202); also, the technology has limited applicability for the epoxidation of terminal olefins like styrene with high ee values, high chemoselectivity and high yield.

The application of enzymes as catalysts in organic synthesis of chiral products is advantageous to obtain high regio-, stereo- and chemospecificity of the reaction (Faber, K., 1997, Biotransformations in organic chemistry, Springer Verlag (Berlin) 3^{rd} ed.).

An alternative precursor with respect to (1S,2R) indene oxide for (-) *cis*-aminoindanol is 1(S)2(R) indanediol. In analogy to (1S,2R) indene oxide, 1(S)2(R) indanediol can be converted in a Ritter reaction to (-) *cis*-aminoindanol. Different biocatalytic routes have been described to yield 1(S)2(R) indanediol with high enantiomeric excess using dioxygenases such as e.g. toluene dioxygenase (Wackett, L.P. et al., 1988, Biochemistry 27: 1360-1367; Lakshman et al., 1998, Synthesis, Sep.: 1352-1356; Buckland, B.C. et al., WO 98/06866 [19.02.98], Allen et al., 1995, J. Chem.Soc., Chem. Commun. 1995: 117-118; Boyd D.R. et al., 1993, J. Chem. Soc., Chem. Commun. 1993: 49-51; Connors N., J. Ind. Microbiol. Biotechnol., 1997, 18: 353-359; Chartrain M. et al., 1998, J. Ferment. Bioeng. 86: 550-558; Treadway S.L. et al., 1999, Appl. Microb. Biotechnol. 51: 786-793; Reddy J. et al., 1999, Appl. Microb. Biotechnol. 51: 614-620).

In these processes, high enantiomeric purities up to > 99% are only obtained in two step reactions using dehydrogenases to further metabolize undesired enantiomers or after chiral crystallization thus lowering the overall yield. The processes use whole cell biocatalysts based on either recombinant cells expressing the necessary genes for desired oxygenases or on knock out mutants of wild type cells carrying the respective oxygenase. Oxygenases involved in these reactions are complex multicomponent enzyme systems with low stability in recombinant form. Wild type cells as biocatalysts often synthesize isomeric oxygenases yielding byproducts (Treadway S.L. et al., 1999, Appl. Microb. Biotechnol. 51: 786-793) thus requiring extensive cell engineering to obtain suitable knock out mutants and bioprocesses specifically designed for these taxa.

Biocatalytic preparation of (1S,2R) indene oxide is possible using fungal haloperoxidases transforming indene to trans-(1S,2S)-bromoindanol as major reaction product. *Trans*(1S,2S)-bromoindanol can subsequently be converted chemically to (1S,2R) indene oxide at high pH (Chartrain et al.,WO 96/36724, [21.11.96]).

In this process, (2R,1R)-bromoindanol is formed among others as a byproduct and degraded by dehydrogenases in the cell, thus increasing the ee value for *trans-*(1S,2S)-bromoindanol to about 80% but at the same time resulting in a low yield of about 30% (Zhang J. et al., 1998, Enz. Microb. Technol., 24: 86-95).

Stereospecific hydrolytic resolution of indene oxide racemates is another biocatalytic way described for the preparation of (1S,2R) indene oxide. Chemical synthesis of racemic indene oxide is straightforward (O'Donell D.J. et al., 1978, J. Org. Chem. 43: 4540). Resolution is achieved using fungal epoxide hydrolases yielding (1S,2R) indene oxide with very high ee values (up to 100%; Zhang et al.1995. J. Ferment. Bioeng. 80: 244-246; Chartrain M.M. et al., WO 96/12818, [02.05.96]). This approach also works for terminal epoxides like styrene oxide using bacterial hydrolases (Archelas A. and Furstoss R., 1998, TibTech 16: 108-116).

The disadvantage of this approach is that the maximal yield is 50%. On the other hand, application of oxygenase enzymes as disclosed in the present invention allows a yield close to 100% with ee values over 98.4%.

Direct biocatalytic epoxidation of indene to indene oxide is described to be catalyzed by rat liver fractions (Francis T.J.R. 1975, Biochem. Soc. Trans. 3: 1244-1246); enantiomeric excess was not reported. 1,2-Epoxyindane was also suggested as an intermediate in the formation of 1-indanol and 1-indanone by the fungus *Pyricularia zingiberi* in addition to various other oxidation products of indene (Nukina M. et al., 1996, Biosci. Biotech. Biochem. 60: 2097-2098).

1,2-Epoxyindane was also suggested as a product of a monooxygenase activity in *Rhodococcus* sp. strain NCIMB 12038, as trans-indane 1,2-diol, the hydrolysis product of indene oxide, was formed from indene by pyruvate or salicylate grown cells. Because of this through-conversion there is no accumulation of 1,2-epoxyindane by this biocatalyst (Allen, C.C.R. et al., 1997, Appl. Environm. Micrbiol. 63: 151-155).

Indene is structurally related to styrene. Vinyl epoxidation of styrene to styrene oxide is described for several oxidative enzymes like cytochrome P-450 monooxygenase (Cox et al., 1996, Appl. Environm. Microbiol. 62: 1471-1474; Fruetel J. et al., 1994, J. Biol. Chem. 269: 28815-28821; Watabe T. et al., 1981, Biochem. Pharmacol. 30: 1695-1698), cytochrome c peroxidase (Miller V.P. et al., 1992, J. Biol. Chem. 267: 8936-8942), chloroperoxidase (Colonna S.N. et al., 1993, Tetrahedron Asamm. 4: 1325-1330; Ortiz de Montellano P.R. et al., 1987, J. Biol. Chem. 262: 11641-11646), soluble methane monooxygenase (Burrows K. et al., 1984, J. Gen. Microbiol. 130: 3327-3333; Colby J. et al., 1977, Biochem. J. 165: 395-402), ammonia monooxygenase (Keener, W.K., 1994, Appl. Environm. Microbiol., 60: 1914-1920), and styrene monooxygenase (e.g. Hartmans S. et al., 1990, Appl. Environm. Microbiol. 56: 1347-1351; Panke S. et al., 1998, Appl.

Environm. Microbiol. 64: 2032-2043; Velasco A. et al., 1998, J. Bacteriol. 180: 1063-1071; DiGennaro P. et al., 1999, Appl. Environm. Microbiol. 65: 2794-2797). Of these different enzymes, high ee-values for the formation of styrene oxide from styrene have only been reported for styrene monooxygenase (ee > 99.5% , Panke S. et al., 1998, Appl. Environm. Microbiol. 64: 2032-2043).

Styrene monooxygenase was supposed to be involved in the formation of 1,2-epoxyindane from indene by mutant M2 derived from *P. putida* S12 by NTG mutagenesis but quantitative data on enantiomeric excess or activity are not given (O'Connor, K.E. 1997, Appl. Environm. Microbiol. 63: 4287-4291). During transformation of styrene, mutant M2 also accumulated styrene oxide from styrene.

In the present invention we disclose methods for the application of diverse styrene monooxygenase enzyme preparations, a.o. to form (1S,2R) indene oxide from indene with ee values > 98.4% in preparative yields.

Epoxidation of vinyl groups has also been described for particulate methane monooxygenase of methylotrophic bacteria (Hou C.-T. et al., US 4,368,267, [11.01.1983]). The epoxidation activity of the enzyme was described to be specific for lower chain length linear olefins, especially propylene and C4 to C6 olefins. The ability of methane monooxygenases to epoxidize vinylaromatic compounds like styrene is also described. A quantification of enzyme activity and stereospecificity is not given. Particulate methane monooxygenase was described to be applicable both in whole cell preparations and in isolated form. On the other hand, activities of such monooxygenase preparations are known to be subject to strong product inhibition in aqueous buffered reaction media (Archelas A. and Furstoss R., 1997, Annu. Rev. Microbiol., 51: 491-525).

The present invention discloses methods to circumvent these limitations by using organic/aqueous emulsions for in situ product extraction thus eliminating any substrate and product inhibition.

The present invention discloses methods to prepare (1S,2R) indene oxide and structurally related derivatives thereof by using monooxygenases like styrene monooxygenase as catalysts. This group of enzymes is defined by their mode of activation of oxygen namely in the form of flavin peroxide. The apo enzyme controls the decay of flavin peroxide and the highly specific insertion of one atom of oxygen in the organic substrate as epoxide functional group. Other oxygenases using other modes of activation (via heme groups or non heme-metal catalysis) do not show this highly specific mode of epoxide formation. Styrene monooxygenase like enzymes are soluble flavin enzymes dependent on nicotinamide cofactors and consist of one or two components. In addition to the mode of oxygen activation, the location in the cytoplasm distinguishes these enzymes significantly from other oxidative enzymes which are membrane associated as they allow easy handling of styrene monooxygenase like enzymes both in recombinant whole cell hosts as well as in isolated form in enzyme reactors. This is due to a higher stability of such enzymes with respect to other oxidative enzymes in reaction media containing oxygen. This allows their use in simple stirred tank reactors with relatively high shear forces as used in examples given hereinafter. Methods for using an isolated complex enzyme like methane monooxygenase including regeneration of cofactors are described in US 4,368,267 (Hou et al., 11.01.1983) but due to low enzyme stability, reaction rates for propylene were only linear up to 15 min and reaction rates for vinylaromatic compounds are significantly lower than with styrene monooxygenase. The present invention discloses methods resulting in high stereospecific activities for vinylaromatic compounds which are stable for 6 hours or more under process conditions.

### Brief description of the drawings

Figure 1 illustrates the reaction involved in the enantiospecific epoxidation of indene (**1**) to (1S,2R) indene oxide (**2**) catalyzed by styrene monooxygenase.

Figure 2 illustrates the substrate spectrum of styrene monooxygenase. The vinylaromatic compounds shown are epoxidized to the corresponding phenyl oxirans in analogy to the formation of styrene oxide from styrene.

### Summary of the invention

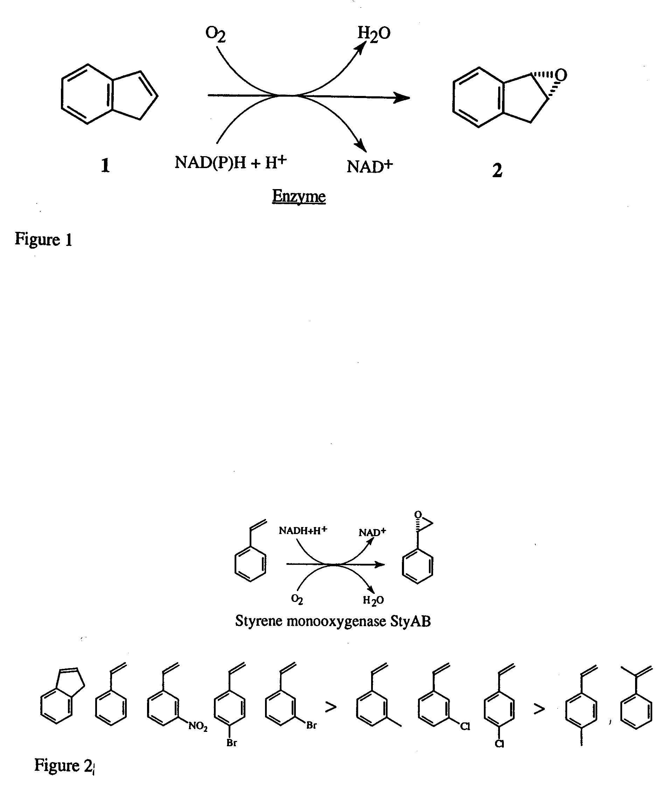

The invention provides a process for the epoxidation of a vinylaromatic compound to a corresponding aromatic epoxide compound which comprises contacting said vinylaromatic compound in a reaction medium with oxygen in the presence of a microbial styrene monooxygenase or styrene monooxygenase like enzyme and optionally isolating the aromatic epoxide compound obtained. In particular, the invention provides a biocatalytic process for the preparation of selected optically active epoxides with high enantiomeric purity. The process disclosed herein comprises a stereoselective insertion of oxygen derived from molecular oxygen catalyzed by a microbial oxygenase enzyme like styrene monooxygenase into olefinic compounds like indene (**1**) or its derivatives as an epoxide function (depicted in Figure 1). In particular, the process is useful for the preparation of (1S,2R) indene oxide (**2**), a key synthon in the synthesis of Crixivan®.

The styrene monooxygenase like enzymes can preferably be used in isolated, partially purified or purified form, or as whole cell preparations using suitable natural strains or recombinant hosts expressing the respective genes of microbial oxygenases like e.g. styrene monooxygenase. Methods for preparing isolated enzymes and recombinant hosts as well as application methods (methods of use) for these systems in organic synthesis are described. In preferred embodiments, a second phase, either solid or liquid, is used during the reaction to extract any aromatic epoxide compound as soon as it is formed in order to prevent product decay and in order to act as substrate reservoir to decrease substrate toxicity and substrate inhibition effects.

Further aspects of the invention will become apparent to those skilled in the art upon consideration of the following detailed description.

### Detailed description of the invention

The term 'microorganism' is used herein in its broadest sense to include not only bacteria, but also yeasts, filamentous fungi, actinomycetes, protozoa, and genetically engineered derivatives of these microorganisms. Preferably, the microorganisms will include bacteria capable of oxidizing styrene and genetically engineered derivatives of these bacteria.

The term 'enzyme preparation' is used to refer to any composition of matter that exhibits the desired oxygenase enzyme activity. The term is used to refer, for example, to living whole cells, dried cells, cell extracts, and refined and concentrated preparations derived from the cells. Enzyme preparations may be either in dry or liquid form. The term also includes the immobilized form of the enzyme, e.g. whole cells of the microorganism or enzyme extracts immobilized or bound to an insoluble matrix by covalent chemical linkages, sorption, and entrapment of the enzyme within a gel lattice having pores large enough to allow the molecules of the substrate and of the product to pass freely, but small enough to retain the enzyme. The term 'enzyme preparation' also includes enzymes retained within hollow fiber membranes, e.g. as disclosed in US 238,649 (Mar. 27, 1972) or in enzyme membrane reactors as disclosed in DE 44 36 149 A1 (18.4.96). Biocatalytic reactions are performed in aqueous medium or in multiphase media possibly containing one or more of the following: a solid phase, an aqueous phase, an organic phase, or a gaseous phase.

The present invention includes the following features:

Microorganisms capable of synthesizing a styrene monooxygenase-like enzyme as defined herein can be isolated from environmental samples using conventional microbiological enrichment techniques in minimal media with a pH of 5 to 9, preferably 7, or using direct plating with styrene or indene as sole carbon source and energy supplied to the microorganisms directly from the agar growth medium in concentrations of preferably 0.1 mM to 5 mM. Alternatively the C-source is supplied via the gas phase from pure styrene or indene or from such substrates diluted in apolar solvents like heptamethylnonane to reduce substrate toxicity. Minimal media agar plates consist of e.g. minimal media with the appropriate C-source like the one described by Dorn et al. (1974, Arch. Microbiol. 99: 61-70) or E2 media (Vogel H.J. and Bonner D.M., 1956, J. Biol. Chem. 218: 97-106). Microorganisms are grown at temperatures between 10 and 50°C, preferably at 25°C. In addition to this, styrene monooxygenase enzymes forming styrene oxide from styrene can be found in a series of microorganisms mineralizing styrene like *Pseudomonas* sp. VLB120, *Pseudomonas fluorescens* ST, *Pseudomonas* sp. Strain Y2, *Pseudomonas putida* S12, *Pseudomonas putida* CA-3, and styrene metabolizing strains as described in Hartmans et al. (Appl. Environm. Microbiol. 56: 1347-1351, 1990) like 124X, S1, S2, S3, S4, S5, S6, S7, S8, S9, S10, S11, S13, S14.

Indene is transformed to 2-indanone by styrene monooxygenase in wild type microorganisms mineralizing styrene via styrene oxide. This was described for *Pseudomonas putida* S12 (O'Connor, K.E. 1997, Appl. Environm. Microbiol. 63: 4287-4291). In the present invention, this reaction was shown to be stoichiometric when using styrene grown cells of *Pseudomonas* sp. VLB120. This is due to high styrene oxide isomerase (SOI) activities transforming indene oxide to 2-indanone. This necessitates either genetic engineering of the respective wild type strains to obtain mutants defective in SOI activity or expression of styrene monooxygenase genes in host microorganisms without enzyme activities degrading indene oxide or other oxides of interest. These microorganisms are preferably *E. coli* or *Pseudomonas putida* KT 2440 derived strains.

Styrene monooxygenase genes can be obtained from respective organisms and expressed in suitable hosts using methods described by Panke et al. (1998, Appl. Environm. Microbiol. 64: 2032-2043). These recombinants can successfully be used as disclosed below as biocatalysts or to obtain enzyme preparations of styrene monooxygenase.

Styrene monooxygenase with high epoxidizing activity can be isolated from either wild type or recombinant microorganisms expressing the respective genes by disrupting cells in a french press, in bead mills, by ultrasonication or by osmotic shock applied to spheroplasts (Witholt, B. et al., 1976, Analyt. Biochem. 74: 160-170). Proteins in cell extracts thus obtained can be either used directly as biocatalyst, or the respective oxygenase can first be enriched, for instance via ion exchange chromatography. This decreases the amount of contaminating proteins in the biocatalyst thus allowing increase of volumetric productivities by reducing reaction volumes.

Protein preparations of styrene monooxygenases and cofactor regeneration systems based on aldehyde dehydrogenases such as e.g. formate dehydrogenase/NAD⁺ can be stabilized using standard immobilization procedures as described by Hartmeier, W. (1986, Immobilized biocatalysts, Springer Verlag, New York) or as disclosed by Reetz T.M. et al in EP 0 676 414 A1 and DE 44 08 152 A1.

Styrene monooxygenases, e.g. StyAB from *Pseudomonas* sp. VLB120, show a broad substrate spectrum for turnover of various styrene derivatives to the corresponding chiral phenyl oxirans (Fig. 2). This allows the application of single enzyme preparations for the production of a range of chiral phenyl oxirans from respective vinylaromatic compounds. Indene is converted to (1S,2R) indene oxide by StyAB with an activity of 80% as compared to styrene as substrate for StyAB. Therefore, all results given for the conversion of styrene to styrene oxide by StyAB are in principle also valid for the conversion of indene to indene oxide by StyAB.

Enzyme preparations of styrene monooxygenase can be used as biocatalysts in aqueous media, preferably in the presence of a second phase, either gaseous, solid or liquid, removing epoxide products *in situ* in order to circumvent product inhibition and product decay due to hydrolysis. Preferably, enzyme preparations are used as catalysts in organic/aqueous emulsions. In these emulsions organic solvents with logP values of 2.5 and higher can be used. Preferably organic solvents are used showing solvent-to-water partition coefficients for vinylaromatic compounds and phenyloxirans over 50, such as dodecane or dioctylphthalate.

Regeneration of nicotinamide cofactors is achieved for whole cells by supplying carbon sources like e.g. glucose or glycerol during the reaction. In the case of the use of isolated styrene monooxygenase, nicotinamide cofactors can be regenerated by methods described by Adlercreutz (Biocatalysis and Biotransformation, 14: 1-30, 1996), for example by *in vitro* regeneration with various enzyme/substrate combinations. Preferably, regeneration of nicotinamide cofactors is achieved enzymatically using formate dehydrogenase and formate as source of reducing equivalents.

Phenyloxiran compounds formed as products can be quantified in the organic solvent phase using normal phase HPLC (eluent: hexane; column: e.g. MN CC250 Nucleosil 100-5 CN) or gas chromatography. In the aqueous phase, phenyl oxirans can be quantified by reversed phase HPLC (eluent acetonitrile/ potassium buffer pH 6 to 8, column with e.g. C-18, ODS2 or C-8 coated silica as matrices). Determination of enantiomer composition is achieved using gas chromatography or normal phase HPLC with β-cyclodextrines as chromatography matrices.

Phenyloxiran compounds formed as products can be purified from aqueous reaction media by solid phase extraction on Amberlite, preferably XAD4 followed by elution with ethanol or methanol. Alternatively, phenyloxirans can be extracted e.g. with ethylacetate after the reaction is completed and recovered by evaporating the solvent. Recovery of phenyloxiran compounds from apolar solvents used in emulsion processes for *in situ* product extraction is achieved preferably via distillation under reduced pressure.

The invention is now illustrated by the following Examples, which describe biocatalytic processes for the oxidation of indene and structurally related phenyloxiran compounds and purification of reaction products. Methods employed in DNA manipulation, basic handling of microbial cells, biocatalysis and biotransformation, protein chemistry, and biochemical engineering are well known to those skilled in the art of genetic engineering, microbiology, purification and handling of proteins, and bioprocess engineering and are described in numerous publications including the following: Sambrook et al.(1989) Molecular cloning, a laboratory manual. 2^{nd} ed. Cold Spring Harbor Laboratory, Cold Spring Harbor, N.Y.; Engel, P.C. (ed.), 1996, Enzymology, LabFax series, Academic Press, San Diego; Price, N.C (ed.). 1996, Proteins, LabFax series, Academic Press, San Diego; Faber K., 1997, Biotransformations in organic chemistry, Springer, New York, 3^{rd} ed; Roberts et al., 1995, Introduction to Biocatalysis using enzymes and micro-organisms, Cambridge University Press; Schlegel, H.-G. 1992, Allgemeine Mikrobiologie, Thieme Verlag, New York; Buchholz K. and Kasche V., 1997, Biokatalysatoren und Enzymtechnologie, VCH, New York; Bailey J.E. and Ollis, D.F., 1986, Biochemical Engineering Fundamentals 2^{nd} ed., McGraw-Hill, Inc. New York.

These examples are not to be taken as limiting in any respect.

### EXAMPLE 1

### Transformation of indene and styrene by styrene monooxygenase in recombinant E. coli

*E. coli* JM101 pStu1, carrying the XhoI/Stu1 DNA fragment of pSPW1 (Panke S. et al., 1998, Appl. Environm. Microbiol. 64: 2032-2043) with *styA* and *styB* from *Pseudomonas* sp. VLB120 was grown on a 2 liter scale in a stirred tank reactor in M9 medium pH 7.2 (37°C), 50 µg/ml Km, 1% glucose to a final cell density of 6.9 gl⁻¹ CDW. After depletion of glucose, 1 mM glycerol was added for regeneration of cellular metabolism and induction of expression of *styA* and *styB* from the lac-promoter on pStul (by overcoming catabolite repression of glucose). Microorganisms started to grow again up to 8.6 gl⁻¹ CDW. Indene was added to a final concentration of 1 mM from a stock solution of 200 mM indene in acetonitrile and transformed to 1,2-indene oxide. After depletion of indene this was repeated two times (after 40 min and 85 min of reaction time). The formation of 1,2 indene oxide was followed by reversed phase HPLC using a RPC18 column (12.5 cm) with a flow rate of 1 ml min⁻¹ and 40% acetonitrile 0.1% H₃PO₄/60% 10 mM potassium phosphate buffer pH 7.5. The total amount of added indene was transformed by styrene monooxygenase with a specific activity of 2.3 U/g CDW to 156 mg indene oxide.

In another experiment, *E. coli* JM101 pStu1 was grown overnight in 200 ml Luria Bertani medium supplemented with 50µg/ml kanamycin at 37°C. Cells were harvested by centrifugation and resuspended in 50 mM Tris/HCl pH 7.6 to a final biomass concentration of 7.7 gCDW l⁻¹. 20 ml of this whole cell preparation of styrene monooxygenase were incubated at 30°C with 1 mM styrene for 30 min. Styrene was transformed to (S) styrene oxide with a yield of 70% and a specific activity of 2.8 U gCDW⁻¹. Incubation of the same batch of *E. coli* JM101 (pStul) with 1 mM indene instead of styrene under the same reaction conditions resulted in formation of 1,2-epoxyindane with 60% yield after 30 min and a specific activity of 3.5 U g CDW⁻¹.

In another typical experiment, styrene was transformed to styrene oxide using whole cells of *E. coli* JM101 (pSPZ10) as the prefered catalyst in a two liquid phase medium (total volume 2 liter) at 30°C. Plasmid pSPZ10 is a pBR322 derivative with *styA* and *styB* (derived from *Pseudomonas* sp. VLB120) under the control of the alkane promoter of *P. oleovorans* alkane degradation genes (Panke S. et al., 1999, Appl. Environm. Microbiol. 65: 2324-2332) conferring kanamycin resistance to host cells. Induced cells overexpressing *styA* and *styB* were prepared in the following way: *E. coli* JM101 (pSPZ10) is grown in a variety of suitable media like complex or minimal growth media, under accepted conditions (temperature between 25°C and 40°C, pH between 6 and 8). In a preferred embodiment, the recombinant bacteria are grown in M9 medium supplemented with thiamine hydrochloride (10µg/ml) and kanamycin (50 µg/ml) and US trace element solution (Panke S. et al., 1999, Appl. Environm. Microbiol. 65: 2324-2332) at 30°C for 12 hours to a cell density of about 2 gCDWl⁻¹. The culture is then supplemented with 0.4% (v/v) US trace element solution and 0.4% (v/v) thiamine hydrochloride (1% w/v). Cells are then grown further to a final cell density of about 5 to 10 gCDWl⁻¹ using a linear feed of 0.5% to 1% (v/v) consisting of 45% (w/v) glucose and 9 gl⁻¹ MgSO₄*7H₂O, adjusted to pH3 with hydrochloric acid. One hour after the feed is started, 0.05% (v/v) dicyclopropylketone or alternatively 1% (v/v) octane is added to the culture medium to induce expression of styrene monooxygenase genes. The inducer was added in 25 to 50% (v/v) of an organic solvent like dodecane, hexadecane, AL240, or preferably dioctylphthalate. The advantage of using dioctylphthalate over other apolar solvents like long chain alkanes as organic phase is that nearly no foam formation is observed during fermentation and that the partition coefficient of vinylaromatic compounds and corresponding oxirans is significantly higher. Styrene was added as a substrate in the organic phase at a concentration of 0.2 M and was oxidized by the growing cells to styrene oxide with a yield of 71% after 7 hours corresponding to a volumetric productivity of 4 g styrene oxide produced per hour and liter aqueous phase.

This experiment was scaled up linearily to the 30 liter scale with 15 liter aqueous phase as described above and 15 liter organic phase consisting of dioctylphthalate.

Scale up was straightforward. As a byproduct 35.9 g phenylethanol were formed, presumably as a product of the reduction of phenylacetaldehyde formed from styrene oxide during the reaction time. This example successfully led to the formation of 388 g (S) styrene oxide (ee > 99.5%) from 676 g of styrene corresponding to a product yield of 49.7% and a styrene turnover of 93%.

In an analogous experiment using the same setup on a 2 liter scale, 20 g indene was transformed into 8.5 g 2-indanone and 11 g of 1S,2R indene oxide.

### EXAMPLE 2

### Analysis of stereoselectivity of the formation of (1S,2R) indene oxide from indene by styrene monooxygenase

*E. coli* (one gram wet weight) cells containing styrene monooxygenase from *Pseudomonas* sp. VLB120 were broken and cell free extract (2.3 mg protein/ml) was prepared as described in Example 3. Indene was transformed at 30°C to 1S,2R indene oxide in a reaction medium containing 1 mM indene, 2 mM NADH, 10 µM FAD, 2% (v/v) acetone, and 460 µg protein from cell free extract in a total of 1 ml 50 mm Tris/HCl pH 7.5. The reaction was stopped after 10 min and indene epoxide was extracted with 1000 µl hexane. The hexane phase was centrifuged for 4 min at 14000 rpm (room temperature), NaSO₄ was added to remove water and removed again by short centrifugation. Enantiomer composition of indene oxide in the hexane phase was determined by using a protocol described by Zhang et al. (1995, J. Ferment. Bioeng. 80: 244-246). In addition, indene was transformed to indene oxide using styrene monooxygenase enriched via anion exchange chromatography on EMD-TMAE material in the same experimental setup as described above.

Enantiomeric excess for 1S,2R indene oxide formed by styrene monooxygenase in cell free extracts was >99%. For 1S,2R indene oxide formed by styrene monooxygenase enriched via anion exchange chromatography, the ee was found to be 98.4%.

### EXAMPLE 3

### Preparation of cell free styrene monooxygenase

In a typical experiment *E. coli* JM101 carrying pSPZ10 is used to prepare large amounts of styrene monooxygenase (see Example 2). Using this protocol, *styAB* is expressed to levels of 2 - 30% of total protein, generally to about 10% StyA with respect to total cell protein. Cells are separated from the culture medium by centrifugation and can be stored frozen for over one year as wet cell pellet after fast freezing in liquid nitrogen, without significant losses of styrene monooxygenase activity at -80°C. Typically 3 g of cell wet weight is resuspendend in Tris/HCl buffer pH 6.5 to 7.5, 1 mM DTT, 1 mM MgCl₂, 10% glycerol and disrupted preferably using a French Press. The cell lysate is ultracentrifuged at 150,000g at 4°C for 45 min. Styrene monooxygenase is recovered in the clear supernatant and can be used as biocatalyst directly. Alternatively, StyAB can be enriched further by a factor of 5 to 10 via anionexchange chromatography either on packed columns (using e.g. EMD-TMAE from Merck, Darmstadt) or by expanded bed chromatography using suitable matrix materials like e.g. Streamline DEAE (Pharmacia). StyAB can be eluted from the column by a linear or stepwise NaCl gradient at a salt concentration of about 100 mM to 250 mM.

Enzyme preparations of StyAB can be lyophylized in 50 mM Tris/HCl buffer pH 7.5 with recoveries up to 95% after rehydration.

### EXAMPLE 4

### Transformation of indene and styrene by styrene monooxygenase in vitro with recycling of NADH cofactor and in situ product extraction

As one representative compound for other phenyloxirans (see Fig. 2) including indene, styrene was transformed by cell free enzyme preparations prepared as described in Example 3 in reaction media containing 50% aqueous phase and 50% organic phase. In a typical experiment, 100 mM styrene in dodecane (50% v/v) is transformed to 57 mM (S) styrene oxide by styrene monooxygenase (StyAB) in the aqueous phase in 4 hours while longer reaction times result in higher yields. This corresponds to a specific activity of styrene oxide formation of 29 U/g protein. StyAB was shown to be stable under reaction conditions up to 8 hours. The aqueous phase consisted in a total of 5 ml of 10 mg/ml protein of cell free extract of *E. coli* JM101 pSPZ10, 20 µM FAD, 0.2 U/ml formate dehydrogenase, 100 mM formate and 1mM NAD⁺. This composition allowed for effective recycling of the NADH cofactor and increased enzyme stability because of the presence of FAD. The cofactor regeneration cycle is started with NAD⁺ instead of NADH because of economical reasons. It can also be started with NADH.

In an analogous reaction indene can be transformed into (1S,2R) indene oxide using solvents like silicon oil as second phase as reservoir of indene and sink for indene oxide.

### EXAMPLE 5

### Transformation of indene by styrene monooxygenase in Pseudomonas sp. VLB120ΔC

The construction of mutants of wild type microorganisms defective in enzymes turning over phenyloxirans of interest is an alternative to the expression of styAB genes in an example of such heterologous hosts, as discussed above. *Pseudomonas* sp. VLB120ΔC is a mutant of wild type strain *Pseudomonas* sp. VLB120 constructed by knocking out styC, the styrene oxide isomerase gene in the wild type. The mutant was constructed and used as biocatalyst as follows: antibiotics were added to the following concentrations for *E. coli* (in parentheses the numbers for *Pseudomonas*) : kanamycin, streptomycin, and spectinomycin 50 µg*mL⁻¹ (100 µg*mL⁻¹); ampicillin 150 µg*mL⁻¹ (piperacillin 50 µg*mL⁻¹). Gene replacement vectors carried an origin of transfer and the counterselectable sacB gene, which conveyed sensitivity to sucrose to the host. They were conjugated from mobilization proficient E. coli S17-1lpir (Simon R. et al., 1983, Bio/Technology 1: 784-791) into *Pseudomonas* sp. strain VLB120. Cells of each strain were taken from overnight LB cultures grown at 30°C. The cells were washed with 1% (w/v) aqueous sodium chloride to remove antibiotics, and an aliquot equivalent to 100 µL of the overnight cultures was spotted on a filter (Type HA, Millipore, Bedford, MA). These filters were placed on LB-agar plates and incubated for 3 h at 30°C. The cells were recovered, washed again in sodium chloride solution, and plated on M9 mineral medium agar plates that contained 0.2% (w/v) citrate as the carbon source, streptomycin, and spectinomycin. Double homologous recombination events were selected for by plating on plates that were prepared as described above and supplemented with 5% (wt/vol) sucrose. For shaking flask experiments various complex and minimal media and conditions can be used. A preferred embodiment used a medium which contained M9* salts, 1 ml l⁻¹ of US trace element solution (Panke S. et al., 1999, Appl. Environm. Microbiol. 65: 2324-2332), and 0.5% (wt/vol) glucose as the carbon source. Inocula for shaking flask experiments were obtained from cultures of the same medium composition. The flasks were incubated at 30°C on a rotary shaker. To induce the styrene oxidation activity, cells received a cell dry weight concentration of 0.1 gl⁻¹ styrene from a 1 M stock in ethanol to a concentration of 1 mM. To ensure continuous styrene supply via the vapor phase, we placed at the same time a sterile tube inside the flask that contained 1 mL of styrene. When strains were investigated for the ability to convert styrene to styrene oxide, the cells were harvested 4 h after induction and were analysed as described in Panke S. et al. (1998, Appl. Environm. Microbiol. 64: 2032-2043).

*Pseudomonas* sp. strain VLB120ΔC was investigated for its styrene monooxygenase induction kinetics in mineral medium that contained glucose as the carbon source. The strain reached a maximum specific activity of 67 U gCDW⁻¹, whereas activities in uninduced cultures remained between 0.2 and 0.7 U gCDW⁻¹. This activity of induced microorganisms enables the formation of indene oxide and other phenyl oxiran derivatives with productivities between 0.5 and 3 gl⁻¹h⁻¹.

### EXAMPLE 6

### Transformation of indene by styrene monooxygenase in Pseudomonas putida SAB

In order to increase the stability of whole cell biocatalysts carrying StyAB, the Palk-StyAB expression cassette was introduced in the chromosome of *Pseudomonas putida* KT2440 using a mini TN5 transposon carrying the cassette giving *Pseudomonas putida* SAB. Specific activities achieved with this recombinant where about 80 U gCDW⁻¹ for formation of styrene oxide from styrene in reaction media like those described in Example 1, containing different carbon sources present in complex and minimal media. Preferably citrate is used as carbon source. This activity of microorganisms enables the formation of indene oxide and other phenyl oxiran derivatives with productivities between 1 and 4 gl⁻¹h⁻¹.

### EXAMPLE 7

### Solid phase extraction mode for preparation of indene oxide

1.5 liter aqueous reaction media including 156 mg 1,2-indene oxide and enzyme preparation (whole recombinant *E. coli* cells) was centrifuged for 20 min at 4°C and 6000g to separate biomass. The clear supernatant was loaded onto 150 g XAD4 (Serva) in a preferred embodiment. XAD-4 was washed sequentially with 300 ml water (step 1), 150 ml 60% acetonitrile (step 2), 50 ml acetonitrile (step 3) and 400 ml acetonitrile (step 4). Initially 85% of total 1,2-indene oxide bound to the column and 34.5% of total 1,2-indene oxide was found in the water of step 1. No 1,2-indene oxide was found in the eluent of step 2. Only 0.8% of total 1,2-indene oxide was found in the eluent of step 3 and 78% of total 1,2-indene oxide was recovered in highly pure form in the eluent of step 4.

This example shows, that solid phase extraction is a suitable method to recover phenyl oxirane compounds from aqueous reaction media.

### EXAMPLE 8

### Recovery and purification of S (+) styrene oxide and (1S, 2R) indene oxide from two liquid phase reaction mixtures

S(+) styrene oxide and (1S,2R) indene oxide prepared in two liquid phase reaction media can be recovered and purified from respective starting materials and byproducts resulting from decay of phenyloxirans like phenylacetaldehyde and 2-indanone by distillation under reduced pressure. In a preferred embodiment, styrene oxide was recovered from dioctylphthalate in good yield at a pressure of 80 mbar at a temperature of 265°C. Indene oxide was recovered from dioctylphthalate at 0.04 mbar and a temperature of 120-150°C.

While the foregoing specification teaches the principles of the present invention, with examples provided for the purpose of illustration, it will be understood that the practice of the invention encompasses all of the usual variations, adaptations, and modifications, as come within the scope of the following claims and its equivalents.

## Claims

1. A process for the epoxidation of a vinylaromatic compound to a corresponding aromatic epoxide compound which comprises contacting said vinylaromatic compound in a reaction medium with oxygen in the presence of a microbial styrene monooxygenase or styrene monooxygenase like enzyme and optionally isolating the aromatic epoxide compound obtained.

2. The process of claim 1, wherein the vinylaromatic compound is contacted under aerobic conditions with cells of a microbial styrene monooxygenase containing organism, a genetically engineered derivative of said cells, or an oxygenase enzyme preparation prepared from said cells or a genetically engineered derivative thereof, wherein said cells, derivative or enzyme preparation exhibit epoxidizing activity.

3. The process of claim 1 wherein the enzyme preparation is derived from a cell free extract of a microbial styrene monooxygenase containing microorganism.

4. The process of any one of claims 1-3 wherein a second phase, either solid or liquid, is used during the reaction to extract any aromatic epoxide compound as soon as it is formed in order to prevent product decay and in order to act as substrate reservoir to decrease substrate toxicity and substrate inhibition effects.

5. The process of any one of claims 1-4 wherein NADH cofactors are recycled during the reaction by reducing equivalents derived from e.g. glucose or glycerol in the case of whole cells containing styrene monooxygenase like enzyme.

6. The process of any one of claims 1-4 wherein NADH cofactors are recycled during the reaction by reducing equivalents derived from e.g. formate and used to regenerate NADH with formate dehydrogenase in the case of isolated monooxygenase.

7. The process of any one of claims 1-6 where FAD and/or glycerol is added to the reaction mixture to stabilize styrene monooxygenase enzyme.

8. The process of any one of claims 1-6 where catalase and/or superoxide dismutase are added to the reaction mixture to stabilize styrene monooxygenase enzyme.

9. The process of any one of claims 1-8 where styrene monooxygenase like enzyme is derived from a microorganism converting styrene to styrene oxide in the course of metabolizing styrene.

10. The process of any one of claims 1-9 wherein said vinylaromatic compound is a non-substituted or substituted vinylaromatic compound which may carry a methyl, halogen or nitro group or any combination of those on the aromatic ring, like e.g. indene, styrene, alpha-methylstyrene, 3-nitro-styrene, 4-bromostyrene, 3-bromostyrene, 3-methylstyrene, 3-chlorostyrene, 4-chlorostyrene and 4-methylstyrene.

11. The process of claim 10 wherein said vinylaromatic compound is indene and is converted to (1S,2R) indene oxide in a stereospecific way without further oxidation of the epoxide.

12. The process of any one of claims 1-11 wherein oxygen is supplied to the reaction in the form of pure oxygen or oxygen in air.

13. The process of any one of claims 1-12 wherein the reaction is conducted as batch process.

14. The process of any one of claims 1-12 wherein the reaction is conducted as continuous process.

15. The process of any one of claims 1-14 wherein the biocatalyst is a knock out mutant of a wild type microorganism containing the monooxygenase gene of interest but being defective in enzyme activities degrading the aromatic epoxy compound of interest.

16. The process of any one of claims 1-15 wherein said biocatalyst is a host microorganism containing said oxygenase gene as stable integrant in its chromosome, which can be expressed to a level of detectable enzyme activity.

17. The process of any one of claims 1-16 wherin the enzyme preparation is obtained by enrichment of cell free extracts via anion exchange chromatography.

18. The process of any one of claims 1-17 wherein said enzyme preparation is lyophilized from tris buffer systems to allow long term storage and simplify handling.

19. The process of any one of claims 1-18 wherein the product is recovered and purified by distillation under reduced pressure.

20. The process of claim 19 wherein the product is styrene oxide and distillation from dioctylphthalate is conducted at 80 mbar and a temperature of 250°C to 270°C.

21. The process of claim 19 wherein the product is indene oxide and distillation from dioctylphthalate is conducted at 0.04 mbar and a temperature of 120°C to 150°C.
